# EUROPEAN PATENT APPLICATION

(11) **EP 3 922 727 A1**
(43) Date of publication of application: **15.12.2021**
(21) Application number: 20179760.2
(22) Date of filing: 12.06.2020
(51) Int. Cl.: C12P 7/22, B01D 15/08, B01D 61/14, B01J 20/20, C12P 7/24, C12P 7/26, C12P 7/40, C12P 17/04

(54) **METHOD FOR SEPARATING BIOMASS FROM A SOLUTION COMPRISING BIOMASS AND AT LEAST ONE AROMA COMPOUND**

(71) Applicant: BASF SE, 67056 Ludwigshafen am Rhein (DE)
(72) Inventor: KAPPIERT, Emiel Jan, 67056 Ludwigshafen (DE); Malisz, Jacek, 67056 Ludwigshafen (DE); CARSTENSEN, Frederike, 67056 Ludwigshafen (DE); HAILER, Anne-Catrin, 67056 Ludwigshafen (DE)
(74) Representative: BASF IP Association

(57) **Abstract**

The present invention relates to a method for separating biomass from a solution comprising biomass and at least one aroma compound comprising providing the solution comprising biomass and aroma compounds, lowering the pH value of the solution below 7 by adding at least one acid to the solution comprising biomass and the at least one aroma compound, adding an adsorbing agent to the solution comprising biomass and aroma compounds and carrying out first membrane filtration so as to separate the biomass from the solution comprising the at least one aroma compound.

## Description

### Technical field

The present invention relates to a method for separating biomass from a solution comprising biomass and at least one aroma compound.

### Background

Attractive smells and flavours are liked by humans for many reasons, and compounds that are perceived as having an attractive smell or flavour are preferred. The cause of this attractive smell or flavour is the perception of aroma compounds, and in consequence aroma compounds have for a long time been of interest.

Natural sources of aroma compounds can be too limited for the demand, and the extraction and / or purification of the aroma compounds form their natural sources often is a laborious process.

Chemical synthesis of some aroma compounds is known, but not all desired aroma compounds can be made efficiently by chemical synthesis alone or require additional treatments. For example, the European patent application EP1081212 discloses a method of purification of furaneol after chemical synthesis using zeolite.

Production by chemical or biotechnological means has been used, but this provides new challenges in the isolation of the desired product.

For example, one or more aroma compound may be produced by means of fermentation providing a solution comprising biomass and at least one aroma compound. Such a solution may also be called fermentation broth.

One examples of aroma compounds produced by fermentation is vanillin. The patents US9115377 and US6133033 disclose the use of Amycolatopsis strains to produce vanillin. The patent application published as CN105132472 discloses the use of Streptomyces psammoticus, while CN1421523 discloses the use of Aspergillus niger. Yeasts have also been used, for example in the international patent application published as WO2007099230

Biomass separation from the fermentation broth from the aroma compound process is the first downstream processing step in the production of aroma compounds. The state-of-the-art technology for this step is centrifugation and or filter press, sometimes with the use of flocculants. However, microfiltration can also be employed and has several advantages in comparison to other separation technologies. To enable a genetically modified organism free product solution, microfiltration is the best option because it can completely retain all non-dissolved solids including genetically modified microorganisms.

### Summary

Membrane filtrations are often used to separate smaller molecules from larger ones in a solution.

The separation of the biomass after fermentative production of aroma compound is usually done at a pH value of 7 to 8, typically around pH 7, by means of an initial centrifugation or filter press and further centrifugations. Sometimes polymeric membranes are used instead.

When membranes are used, however, the membrane performance is rather low and the permeate contains a lot of proteins and colour components, which have to be removed in the following steps leading to an elaborate downstream process, high product yield losses and some quality problems.

Typically, after these initial steps of biomass separation from fermentation broths, the next step carried out is an ultrafiltration completed typically with 10 kDa polyethersulfone membranes, yet not all proteins and polysaccharides can be separated by this. The ultrafiltration permeate is hence sent to an active carbon column to decolorize the solution and achieve an APHA value of below 1000. The decolourization in the active carbon column is a rather tedious process and it is often necessary to use around 14% weight/weight of active carbon in relation to the initial amount of fermentation broth. This step leads to high product losses and necessitates huge active carbon columns.

It was therefore an object of the invention to avoid the abovementioned disadvantages. In particular, a method should be provided that is suitable to enhance the performance of separating biomass from a solution comprising biomass and at least one aroma compound and to reduce the amount of proteins in and the colour of the filtration permeate.

According to the present invention, this object is solved by a method for separating biomass from a solution comprising biomass and at least one aroma compound, comprising:
- providing the solution comprising biomass and aroma compounds,
- lowering the pH value of the solution below 7, preferably below pH 5.5 or less by adding at least one acid to the solution comprising biomass and the at least one aroma compound,
- adding an adsorbing agent to the solution comprising biomass and aroma compounds, and
- carrying out a membrane filtration also called herein the first membrane filtration and typically being a microfiltration or ultrafiltration so as to separate the biomass from the solution comprising the at least one aroma compound. Preferably, the sequence of method steps is the one given in the previous sentence.

According to the method of the present invention, it was surprisingly found, that the membrane performance can be significantly increased, and removal of proteins can be significantly improved when the pH value of the solution is lowered below 7. Further, it was found that membrane performance increases further and the colour of the permeate can be significantly reduced to values below the required specification when an adsorbing agent is added to the solution before any membrane filtration. Also advantageously, the needed amount of adsorbing agent like active carbon is much lower as compared to the known methods, and also the required time for decolourization is much shorter than in known methods, when the membrane filtration is done after the pH value has been set to the desired target value below pH 7 and at least on adsorbing agent has been added.

Preferably, the adsorbing agent is active carbon. Active carbon, also known as activated carbon or activated charcoal, is a preferred adsorbing agent as it is of low cost, available in large quantities, easy to handle and safe to use in foodstuff.

It is beneficial to the methods of the invention that the pH value of the solution comprising biomass and one or more aroma compound, one or more disaccharide and / or one or more monosaccharide is below pH 7.0 when the first membrane filtration is performed, and more preferably when the adsorbing agent is added. Hence, since pH values of fermentation broth are typically at or above pH 7.0, the pH value is lowered by the addition of at least one acid as needed to achieve the target pH value. In case the pH value of the solution comprising biomass and one or more aroma compound, one or more disaccharide and / or one or more monosaccharide is already below pH 7.0 at the start, at least one acid may be used for setting the pH value stably below pH7.0 as needed. Also, preferably, the pH value of the solution is set to a pH value of 5.5 or below, before any membrane filtration is started. Preferably the pH value is lowered to a target pH value in the range of 3.0 to 5.5, more preferably the range of 3.5 to 5, wherein the ranges given include the given numbers. In an even more preferred embodiment, the pH value of the solution is set to pH 3.5 or above, but not higher than pH 4.5 and most preferably the pH value is set to a value in the range of and including 4.0 to 4.5. To this end, at least one acid is added to the solution. Said at least one acid is, more preferably, an acid selected from the group consisting of H₂SO₄, H₃PO₄, HCl, HNO₃ and CH₃CO₂H. Basically, any acid may be used. Nevertheless, these acids are usually easy to handle.

Said adsorbing agent, preferably active carbon, is typically added in an amount in the range of 0.25 % to 3 % by weight, preferably in the range of 0.5 % to 2.5 % by weight and more preferably in the range of 0.75 % by weight to 2.2 % by weight and even more preferably in the range of 1.0 % to 2.0 % by weight or 0.5 to 1.5 % by weight, wherein the percentage values are on a weight of adsorbing agent per weight of solution basis. Thus, a rather small amount of said adsorbing agent, preferably active carbon, is sufficient to reduce the colour number below the upper bound specification, which is preferably 1000 APHA. This allows for significant reduction of active carbon consumption as well as for significant reduction of product losses in comparison to the active carbon column. In one embodiment one or more adsorbing agents are added in an amount suitable to bind - in increasing order of preference - at least 50%, 55 %, 60 %, 65 %, 70 %, 75 %, 80 %, 90 %, 92 %, 94 %, 95 % or more of the colour components and / or the protein in the starting solution comprising biomass and / or polysaccharides and / or proteins and / or nucleic acids like DNA or RNA that may be present. Further, said adsorbing agent, preferably active carbon, is typically added as a powder having a particle size distribution with a diameter d50 in the range of 2 µm to 25 µm, preferably in the range of 3 µm to 20 µm and more preferably in the range of 3 µm to 7 µm, and even more preferably in the range of 5 µm to 7 µm. The d50 value is determined with standard procedures. Particle sizes in this size range reduce the risk of abrasion of the membrane. Moreover, said adsorbing agent, preferably active carbon, is yet preferably added as a suspension of the powder in water. This facilitates handling of the adsorbing agent as the suspension of the powder may better mix with the suspension comprising biomass and the aroma compound. The adding said adsorbing agent, preferably active carbon, to the solution is, typically, carried out after adding the at least one acid to the solution. Unexpectedly, the colour reduction and protein reduction are much better, when the pH value is adjusted first and then the adsorbing agent or at least the majority of the adsorbing agent is added subsequently. It is possible to add said adsorbing agent, preferably active carbon, to the fermentation broth before adding the at least one acid to the solution.

In another variant, the pH value of the solution is lowered to 5.5, more preferably to 5.0 and even more preferably to 4.5 by the addition of at least one of the suitable acids, and then adsorbing agent, preferable active carbon, and further acid is added until the desired final pH value is achieved.

Also, some of the adsorbing agent may be added before any acid is added to lower the pH value, followed by the addition of more adsorbing agent after the pH value has been set to the target value below pH 7.0.

Preferably, said solution comprising biomass and one or more fine chemicals, preferably one or more aroma compounds, typically is a fermentation broth, obtained by cultivation of one or more types of cells, preferably bacteria, plant or yeast cells, more preferably bacteria, even more preferably *Escherichia coli, Amycolatopsis sp or Rhodobacter sphaeroides*, in a cultivation medium, preferably a cultivation medium comprising at least one carbon source, at least one nitrogen source and inorganic nutrients. Thus, sufficient amounts of said fine chemical(s), preferably aroma compound(s), may be produced with cost efficient methods.

Said microfiltration or ultrafiltration of the first membrane filtration step is typically carried out as cross-flow microfiltration or cross-flow ultrafiltration. Thus, the filtration efficiency may be enhanced. Said cross-flow microfiltration or cross-flow ultrafiltration includes a cross-flow speed above 0.2 m/s, preferably in the range of 0.5 m/s to 6.0 m/s, more preferably in the range of 2.0 m/s to 5.5 m/s and even more preferably in the range of 2.8 m/s to 4.5 m/s, and most preferably in the range of 3.0 m/s to 4.0 m/s if ceramic mono- and multi-channel elements are used. In another embodiment, the cross-flow speed is equal to or below 3.0 m/s. In case that a polymeric membrane is used for the first membrane filtration, cross-flow speeds of 2 m/s or less can be used; cross-flow speeds in the range of 0.5 m/s to 1.7 m/s are preferably used, but even cross-flow speeds of 0.5 m/s or less may be used. In another preferred embodiment, the cross-flow speed is not more than 1.7 m/s, 1.6 m/s, 1.5 m/s, 1.4 m/s, 1.3 m/s, 1.2 m/s, 1.1 m/s or 1.0 m/s if a polymeric membrane is used. Thus, the filtration speed may be optimized when compared to a filtration process without including a pH value adjustment and addition of an adsorbing agent. By doing so, wear and tear on and/or energy consumption of the membrane filtration equipment can be reduced by operating at lower cross-flow speed compared to previously known methods, while resulting in good separation.

Said first membrane filtration, preferably a microfiltration or ultrafiltration is, typically, carried out at a temperature of the solution in the range of 4 °C to 55 °C, preferably in the range of 10 °C to 50 °C and more preferably in the range of 30 °C to 40 °C. Thus, the temperature during said filtration step may be the same as during fermentation which further improves the membrane performance and decreases viscosity of the solution comprising biomass and aroma compound. Yet, the first membrane filtration is, also preferably, carried out by means of a ceramic microfiltration membrane or ceramic ultrafiltration membrane having a pore size in the range of 20 nm to 800 nm, preferably in the range of 40 nm to 500 nm and more preferably in the range of 50 nm to 200 nm. It is also possible to use multi-layered membranes that are engineered to have improved abrasion resistance, e.g. 400 nm and 200 nm and 50 nm pore size layers of Al₂O₃. Thus, sufficient amounts of proteins and polysaccharides may be removed in order to comply with the desired specification. Also typically, first membrane filtration is carried out by means of a polymeric microfiltration membrane or polymeric ultrafiltration membrane having a cut-off above or equal to 4 kDa, preferably in the range of 10 kDa to 200 nm, more preferably in the range of 50 kDa to 200 nm and even more preferably equal to or above 100 kDa. In another preferred embodiment the cut-off is 100nm or less. Thus, sufficient amounts of proteins and polysaccharides may be removed in order to comply with the desired specification.

The polymeric material of the polymeric microfiltration membrane or polymeric ultrafiltration membrane is, preferably, at least one polymeric material selected from the group consisting of: polyethersulfone, polysulfone, polypropylene, polyvinylidene fluoride, polyacrylonitrile, polyvinylidene fluoride. Modified polymeric materials can also be used, for example hydrophilized polyethersulfone.

The ceramic material of the ceramic microfiltration membrane or ceramic ultrafiltration membrane is, preferably, at least one ceramic material selected from the group consisting of: TiO₂, ZrO₂, SiC and Al₂O₃.

The first membrane filtration, preferably microfiltration or ultrafiltration is, typically, carried out after a predetermined time after the adsorbing agent, preferably active carbon, has been added to the solution. This allows to provide an adsorption time during which colour components are adsorbed. Said predetermined time is at least 2 min, preferably at least 10 min and more preferably at least 20 min. Thus, the adsorption of colour components is rather quick.

The method may, preferably, further comprise carrying out a second or further membrane filtration, preferably an ultrafiltration, using the solution essentially free of biomass obtained by the microfiltration or ultrafiltration of the first membrane filtration and comprising one or more aroma compound, one or more disaccharides and / or one or more monosaccharides, preferably comprising the majority of these saccharides from the starting solution, e.g. the fermentation broth, that also comprised the biomass . Preferably, the second membrane filtration is done with the permeate of the first membrane filtration and with a membrane having a lower cut-off than the first membrane. Thus, an advantageous further processing of the permeate obtained by the first membrane filtration is realized. The second membrane filtration is, typically, an ultrafiltration carried out by means of an ultrafiltration membrane, preferably, at least partially made of a polymeric material, and having a cut-off in the range of 1 kDa to 10 kDa, preferably in the range of 2 kDa to 10 kDa and more preferably in the range of 4 kDa to 5 kDa. Polymeric membranes typically offer the advantage over tight ceramic membranes that they are more robust and less expensive.

The second membrane filtration may be performed with a ceramic membrane of 1 to 25 kDa cut-off. In a further embodiment it is preferable that the membrane is at least partially made of a polymeric material. Said polymeric material is, more preferably, at least one polymeric material selected from the group consisting of: polyethersulfone, polysulfone, polyacrylonitrile, cellulose acetate. Said second membrane filtration is, typically, carried out after adjusting the temperature of the solution to temperatures of below 20, preferably at a temperature of the solution being in the range of 4 °C to 15 °C, preferably in the range 8 °C to 13 °C and more preferably in the range 8 °C to 12 °C.

In a preferred embodiment, the first membrane filtration employed in the inventive methods includes two or preferably three steps as will be explained in further detail below. The first step includes a first diafiltration having a diafiltration factor DF (amount of diafiltration water = starting amount of fermentation broth x diafiltration factor) ranging from 0.5 or less to 3 or above. For example, for 2'FL comprising solutions it was advantageous to have a DF of 0.5 while for other aroma compound molecules values of 3 proved to be better if a concentration step was to follow. During diafiltration, the amount of water or a suitable aqueous solution added is identical to the amount of permeate discharged. In a batch wise diafiltration, the volume in the feed vessel is thus kept constant. The second step includes concentrating of the fermentation broth preferably with a factor 2 or more by stopping the feed of diafiltration water and the level will decrease down to the target value (target value = volume or mass at the beginning of the fermentation broth / concentrating factor). Optionally, the subsequent third step includes a second diafiltration. By means of these three steps a lower dilution of the product within the permeate and an increased yield of ≥ 95% are realized. By increasing the factor of the second diafiltration, the yield may even be further increased. However, the dilution of the product will also increase. The permeate then typically is the combination of all solutions passing through the membrane in these three steps. In a batch process each step produces a permeate fraction in a time-separated manner, that can be collected in one vessel for mixing, or processed separately. In a continuing process, each of the three steps produces a permeate fraction not in a time separated, and these fractions can be combined to form the permeate combined or treated separately if desired.

Optionally the first step of the first membrane filtration may be repeated one or more times, before the second step of concentration is done. Optionally, the second step may be performed, or it may be skipped if concentrating the solution is not desirable. This is useful when the fermentation broth has a high viscosity and or very high biomass content, for example.

Optionally the first step may be skipped and alternatively the second step is done without the first step, so that first a concentration of the fermentation broth is done while creating permeate, and then a diafiltration of the last step is done by feeding water or aqueous solutions to the solution comprising biomass and one or more aroma compounds.

A further embodiment is the inventive apparatus suitable to perform the methods of the invention.

Further features and embodiments of the invention will be disclosed in more detail in the subsequent description, particularly in conjunction with the dependent claims. Therein the respective features may be realized in an isolated fashion as well as in any arbitrary feasible combination, as a skilled person will realize. The embodiments are schematically depicted in the figures. Therein, identical reference numbers in these figures refer to identical elements or functionally identical elements.

### Detailed description

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "particularly", "more particularly", "specifically", "more specifically", "typically", "more typically", "preferably", "more preferably" or similar terms are used in conjunction with additional / alternative features, without restricting alternative possibilities. Thus, features introduced by these terms are additional / alternative features and are not intended to restrict the scope of the claims in any way. The invention may, as the skilled person will recognize, be performed by using alternative features. Similarly, features introduced by "in an embodiment of the invention" or similar expressions are intended to be additional / alternative features, without any restriction regarding alternative embodiments of the invention, without any restrictions regarding the scope of the invention and without any restriction regarding the possibility of combining the features introduced in such way with other additional / alternative or non-additional / alternative features of the invention.

As used herein, the term "biomass" refers to the mass of biological material comprised in the solution. Typically, said biological material in accordance with the present invention are one or more types of prokaryotic or eukaryotic organisms, or parts thereof, such as cell walls, proteins, phospholipids, cell membranes, polynucleotides and other large organic compounds produced by the microorganism.

In a particular preferred embodiment, biomass refers to one or more biological organisms, more preferably the one or more organism is a bacterium or a fungal or a plant cell or non-human animal cell. In a preferred embodiment, the one or more organism is a bacterial cell selected from a) the group of Gram negative bacteria, such as Rhodobacter, Agrobacterium, Paracoccus, or Escherichia; b) a bacterial cell selected from the group of Gram positive bacteria, such as Bacillus, Corynebacterium, Brevibacterium, Amycolatopis; c) a fungal cell selected from the group of Aspergillus (for example Aspergillus niger), Blakeslea, Peniciliium, Phaffia (Xanthophyllomyces), Pichia, Saccharamoyces, Kluyveromyces, Yarrowia, and Hansenula; or d) a transgenic plant or culture comprising transgenic plant cells, wherein the ocell is of a transgenic plant selected from Nicotiana spp, Cichorum intybus, lacuca sativa, Mentha spp, Artemisia annua, tuber forming plants, oil crops and trees; e) or a transgenic mushroom or culture comprising transgenic mushroom cells, wherein the microorganism is selected from Schizophyllum, Agaricus and Pleurotisi. More preferred organisms are microorganism belonging to the genus Escherichia, Saccharomyces, Pichia, Amycolatopsis, Rhodobacter, and even more preferred those of the species *E.coli, S.cerevisae, Rhodobacter sphaeroides* or *Amycolatopis sp.,* for example but not limited to *Amycolatopsis mediterranei, for example the strain NCIM 5008,Streptomyces setonii, Streptomyces psammoticus,* and for example but not limited to *Amycolatopsis sp* strains IMI390106 , Zyl 926, ATCC39116, DSM 9991, 9992 or Zhp06.

More preferably, the said biomass comprises microorganisms, even more preferably genetically modified *microorganisms,* which are cultivated in a cultivation medium, preferably a cultivation medium comprising at least one carbon source, at least one nitrogen source and inorganic nutrients.

In a further embodiment, the methods of the invention are applied to separate aroma compounds, disaccharides and monosaccharides produced from macromolecular biomass, such as wood, straw, stalks and other plant material containing lignin, cellulose and/or starch, or from macromolecular biomass or animal or microbial origin, such as chitin containing substances, polysaccharides and the like from the remainders of said macromolecular biomass.

The easiest way to assess the success of separating the biomass and the aroma compound(s), disaccharide(s) and/ or monosaccharide(s) is to monitor that the permeate of the first membrane filtration is optically clear. Unsuccessful separation will result in biomass being detected in the optical check of the permeate, and the presence of adsorbing agent like black active carbon in the permeate will also easily be detected in the optical check and indicate a leak or failure of the membrane filtration equipment.

As used herein, the term "aroma compound" refers to any substance that is an odorant, aroma, fragrance, or flavour, and preferably is a chemical compound that has a smell or odour. Preferably an aroma compound is an organic compound typically with less than 300 as a molecular weight; and more preferably the aroma compound is a polar aroma compound, even more preferably is selected from the list of furaneol, benzoic acid, phenylethanol, raspberry ketone, pyrazines, vanillin, vanillyl alcohol and vanilla glycoside, and yet even more preferably it is selected from vanillin, vanillyl alcohol and vanilla glycoside.

In one embodiment the methods of the invention refer to methods that produce a purified precursor of the final aroma compound rather than the finished aroma compound, wherein the precursor has been produced by fermentation and is present in the fermentation broth subjected to the inventive treatment. The methods of the invention, apparatus of the invention may as part of this invention be applied to such precursor molecules that after the purification by the inventive methods or in the inventive apparatus are then transformed into the final aroma compound by one or more enzymatic and/or chemical steps. Therefore, in one embodiment any reference to an aroma compounds may be understood to refer to a precursor of said aroma compound.

As used herein, the term "disaccharide" refers to a saccharide consisting of two monosaccharides, for example lactose that consists of a glucose and a galactose moiety, or saccharose that is made from one glucose and one fructose molecule.

As used herein, the term "monosaccharide" refers to a simple sugar, preferably a sugar molecule comprising 5 or 6 carbon atoms, for example glucose, fructose, galactose or fucose.

The term "adsorbing agent" as used herein refers to an element configured to provide the adhesion of atoms, ions or molecules from a gas, liquid or dissolved solid to a surface. The term "adhesion" refers to the tendency of dissimilar particles or surfaces to cling to one another. Preferably, the adsorbing agent is configured to provide adhesion for colour components. Preferably, the adsorbing is active carbon.

As used herein, the term "microfiltration" refers to a type of physical filtration process where a fluid comprising undesired particles, for example contaminated fluid is passed through a special pore-sized membrane to separate microorganisms and suspended particles from process liquid, particularly larger bacteria, yeast, and any solid particles. Microfiltration membranes haves a pore size of 0.1 µm to 10 µm. Thereby, such membranes have a cut-off for a molecular mass of more than 100000 kDa.

As used herein, the term "ultrafiltration" refers to a type of physical filtration process where a fluid comprising undesired particles, for example contaminated fluid is passed through a special pore-sized membrane to separate microorganisms and suspended particles from process liquid, particularly bacteria, macromolecules, proteins, larger viruses. Ultrafiltration membranes have typically a pore size of 2 nm to 100 nm and have a cut-off for a molecular mass of 2 kDa to 250000 kDa. The principles underlying ultrafiltration are not fundamentally different from those underlying microfiltration. Both of these methods separate based on size exclusion or particle retention but differ in their separation ability depending on the size of the particles.

According to the present inventive methods, first membrane filtration is carried out preferably by means of a polymeric microfiltration membrane or polymeric ultrafiltration membrane having a cut-off equal to or above 4kDa, preferably in the range of 10 kDa to 200 nm, more preferably in the range of 50 kDa to 200 nm and even more preferably in the range of 50 kDa to 100nm. Further, said second membrane filtration is preferably carried out by means of an ultrafiltration membrane having a cut-off in the range of 1kDa to 10 kDa, preferably in the range of 2 kDa to 10 kDa and more preferably in the range of 4 kDa to 5 kDa.

The cut-off of a filtration membrane typically refers to retention of 90 % of a solute of a given size or molecular mass, e.g. 90% of a globular protein with x kDa are retained by a membrane with a cut-off of x kDa. These cut-off values can be measured for example by the use of defined dextranes or polyethylene glycols and analyzing the retentate,the permeate and the original solution also called feed with a GPC gel permeation chromatography analyser using methods and parameters common in the art.

As used herein, the term "cross-flow filtration" refers to a type of filtration where the majority of the feed flow travels tangentially across the surface of the filter, rather than into the filter, at positive pressure relative to the permeate side. The principal advantage of this is that the filter cake which can blind the filters in other methods is not building up during the filtration process, increasing the length of time that a filter unit can be operational. It can be a continuous process, unlike batch-wise dead-end filtration. For large scale applications, a continuous process is preferable. This type of filtration is typically selected for feeds containing a high proportion of small particle size solids where the permeate is of most value because solid material can quickly block (blind) the filter surface with dead-end filtration. According to the present disclosure, said cross-flow microfiltration or cross-flow ultrafiltration includes a cross-flow speed in the range of 0.5 m/s to 6.0 m/s, preferably in the range of 2.0 m/s to 5.5 m/s and more preferably in the range of 3.0 m/s to 4.5 m/s. In case of a membrane made of ceramics, the cross-flow speed may be higher than in case of a membrane made of a polymeric material depending on the respective geometry of the membrane. For example, in case of a flat polymeric membrane such as a polymeric membranes in flat sheet modules, the cross-flow speed is 0.5 m/s to 2.0 m/s and preferably 1.0 m/s to 1.7 m/s. and more preferably 1.0 to 1.5 m/s. Depending on the particular set-up and the particular solution comprising the biomass even cross-flow speeds of 1.0 m/s or less may be used in some cases, yet the filtration may turn into a dead end filtration when the cross-flow speeds are too low.

The term "cut-off" as used herein refers to the exclusion limit of a membrane which is usually specified in the form of MWCO, molecular weight cut off, with units in Dalton. It is defined as the minimum molecular weight of a solute, for example a globular protein that is retained to 90% by the membrane. The cut-off, depending on the method, can be converted to so-called D90, which is then expressed in a metric unit.

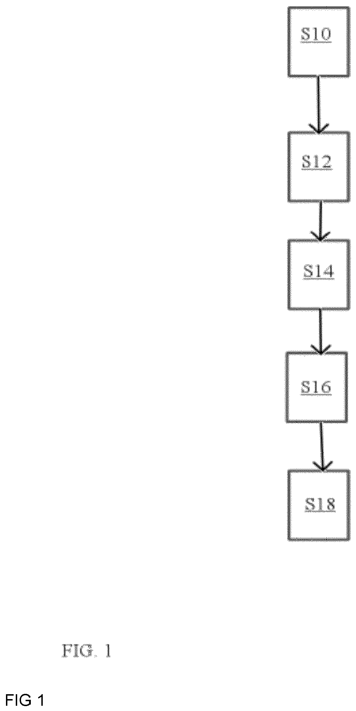

In a first step (Fig. 1, step S10), a solution comprising at least one aroma compound produced in a fermentative process is provided. Said at least one aroma compound comprises aroma compound, preferably a polar aroma compound, even more preferably ... vanillin or a precursor thereof. Preferably, said solution comprises biomass as well as at least one aroma compound and is obtained by cultivation of one or more types of cells in a cultivation medium. Thus, said solution may also be called fermentation broth in a preferred embodiment. The cultivation medium is preferably a cultivation medium comprising at least one carbon source, at least one nitrogen source and inorganic nutrients. More preferably, the fermentation broth or solution comprising biomass and the at least one aroma compound is obtained by microbial fermentation, preferably aerobic microbial fermentation. A microorganism capable of producing the aroma compound may be a yeast or a bacterium or a plant cell or an animal cell, for example from the group consisting of the genera Escherichia, Klebsiella, Helicobacter, Bacillus, Lactobacillus, Streptococcus, Amycolatopsis, Rhodobacter, Lactococcus, Pichia, Saccharomyces and Kluyveromyces. The aqueous nutrient medium comprises at least one carbon source (e.g. glycerol or glucose) which is used by the microorganism for growth and/or for biosynthesis of the aroma compound. In addition, the nutrient medium also contains at least one nitrogen source, preferably in the form of yeast extract or an ammonium salt, e.g. ammonium sulphate, ammonium phosphate, ammonium citrate, ammonium hydroxide etc., which is necessary for the growth of the microorganisms. Other nutrients in the medium include e.g. one or several phosphate salts as phosphor source, sulphate salts as sulphur source, as well as other inorganic or organic salts providing e.g. Mg, Fe and other micronutrients to the microorganisms. In many cases, one or more vitamins, e.g. thiamine, has to be supplemented to the nutrient medium for optimum performance. The nutrient medium may optionally contain complex mixtures such as yeast extract or peptones. Such mixtures usually contain nitrogen-rich compounds such as amino acids as well as vitamins and some micronutrients.

The nutrients can be added to the medium at the beginning of the cultivation, and/or they can also be fed during the course of the process. Most often the carbon source(s) are added to the medium up to a defined, low concentration at the beginning of the cultivation. The carbon source(s) are then fed continuously or intermittently in order to control the growth rate and, hence, the oxygen demand of the microorganisms. Additional nitrogen source is usually obtained by the pH control with ammonia (see below). It is also possible to add other nutrients mentioned above during the course of the cultivation.

In some cases, a precursor compound is added to the medium, which is necessary for the biosynthesis of the aroma compound. The precursor compound may be added to the medium at the beginning of the cultivation, or it may be fed continuously or intermittently during the cultivation, or it may be added by a combination of initial addition and feeding.

The cells are cultivated under conditions that enable growth and biosynthesis of the aroma compound in a stirred tank bioreactor. For aerobic conditions a good oxygen supply in the range of 50 mmol O₂/(l*h) to 180 mmol O₂/(l*h) to the microbial cells is essential for growth and biosynthesis, hence in such a setting the cultivation medium is aerated and vigorously agitated in order to achieve a high rate of oxygen transfer into the liquid medium in case aerobic conditions are desired, and optionally, the air stream into the cultivation medium may be enriched by a stream of pure oxygen gas in order to increase the rate of oxygen transfer to the cells in the medium.

Anaerobic conditions are herein defined as conditions without any oxygen or in which substantially no oxygen is consumed by the cultured cells, in particular a microorganism, and usually corresponds to an oxygen consumption of less than 5 mmol/l.h, preferably to an oxygen consumption of less than 2.5 mmol/l.h, or more preferably less than 1 mmol/l.h.

Oxygen-limited conditions are defined as conditions in which the oxygen consumption is limited by the oxygen transfer from the gas to the liquid. The lower limit for oxygen-limited conditions is determined by the upper limit for anaerobic conditions, i.e. usually at least 1 mmol/l.h, and in particular at least 2.5 mmol/l.h, or at least 5 mmol/l.h. Some organisms are grown in micro-aerob conditions as a special variant of oxygen limited conditions. In such cases substantially more than 5 mmol/l.h are used, but not enough to arrive at full aerobic conditions. The upper limit for oxygen-limited conditions is determined by the lower limit for aerobic conditions, i.e. less than 100 mmol/l.h, less than 50 mmol/l.h, less than 20 mmol/l.h, or less than to 10 mmol/l.h, depending on the organisms and the set-up like growth temperature, growth stage and the like. For example, the organism may be kept at dissolved oxygen levels between 10 % and 40%, for example at 35 % and then after some time reduced to 15%.

Aerobic conditions are conditions in which a sufficient level of oxygen for unrestricted growth is dissolved in the medium, able to support a rate of oxygen consumption of at least 10 mmol/l.h, more preferably more than 20 mmol/l.h, even more preferably more than 50 mmol/l.h, and most preferably more than 100 mmol/l.h.

Whether conditions are aerobic, anaerobic or oxygen-limited is dependent on the conditions under which the method is carried out, in particular by the amount and composition of ingoing gas flow, the actual mixing/mass transfer properties of the equipment used, the type of micro-organism used and the micro-organism density.

Generally, the temperature is at least 0°C, in particular at least 15°C, more in particular at least 20°C. A desired maximum temperature depends upon the enzymes producing the aroma compound and the host cell used. Depending on the cells and/or the enzymes producing the aroma compound used, the temperature is 70 ° or less, preferably 50 °C or less, more preferably 40 °C or less, in particular 37 °C or less. Organisms like Thermus thermophilus have a temperature optimum for growth between 49°C and 72°C, Escherichia coli of about 37 °C, and many fungal microorganisms like yeasts and bacterial microorganisms like Rhodobacter spaeroides have temperature optima around 30°C. In case of a fermentative process, the incubation conditions can be chosen within wide limits as long as the cells show sufficient activity and/or growth. This includes pH ranges, temperature ranges and aerobic, oxygen-limited and/or anaerobic conditions.

In one embodiment the cultivation is carried out at 24°C to 41°C, preferably 28°C to 40°C, more preferably at 30°C or more. Depending on the organism used a temperature of around 30°C or in the range of 32°C to 39°C is used. The pH value is set at 6.2 to 7.2, preferably by automatic addition of NH₃ (gaseous or as an aqueous solution of NH₄OH).

In some cases, the biosynthesis of the aroma compound needs to be induced by addition of a chemical compound, e.g. Isopropyl β-D-1-thiogalactopyranoside (IPTG) for example as in the European patent application published as EP 2 379 708. The inducer compound may be added to the medium at the beginning of the cultivation, or it may be fed continuously or intermittently during the cultivation, or it may be added by a combination of initial addition and feeding.

Subsequently, the method of the invention proceeds to the adjustment of the pH value in a second step (Fig. 1, step S12). In said step, typically the pH value of the solution is lowered by adding at least one acid to the solution comprising biomass and the at least one aroma compound. The pH value of the solution is lowered to a target pH value preferably in the range of 3.0 to 5.5, more preferably in the range of 3.5 to 5 and even more preferably in the range of 4.0 to 4.5, such as 4.0 or 4.1. Said at least one acid is an acid selected from the group consisting of H₂SO₄, H₃PO₄, HCl, HNO₃ (preferably not in concentrated form) and CH₃CO₂H, or any other acid considered safe in production of food or feed; preferably the acid is selected from the group consisting of H₂SO₄, H₃PO₄, HCI and CH₃CO₂H. A mix of these acids may be used in one embodiment instead of a single of these acids.

Further, in another embodiment of the method of the invention, if the solution comprising biomass and the at least one aroma compound, and optionally at least one disaccharide or at least one monosaccharide already has a pH value below 7, preferably below pH 5.5, more preferably equal to or below pH 5.0 and even more preferably equal to or below pH 4.5, there will be no addition of any of these acids, and step S12 may be skipped and the methods of the invention for such solutions continues with Step S14.

The method then proceeds to the next step (Fig. 1, S14). In said step, one or more adsorbing agent is added to the solution comprising biomass and the at least one aroma compound. Preferably, the adsorbing agent is active carbon. Said adsorbing agent, preferably active carbon, is added in an amount in the range of 0.5 % to 3 % by weight, preferably in the range of 0.6 % to 2.5 % by weight and more preferably in the range of 0.7 % to 2.0 % by weight, such as 1.5 %. In this respect, it has to be noted that the smaller the particles of the adsorbing agent are, the better the adsorption characteristics are. Said adsorbing agent, preferably active carbon, is added as a powder having a particle size distribution with a diameter d50 in the range of 2 µm to 25 µm, preferably in the range of 3 µm to 20 µm and more preferably in the range of 3 µm to 7 µm such as 5 µm. More preferably, said adsorbing agent, preferably active carbon, is added as a suspension of the powder in water. Preferably, adding said adsorbing agent, preferably active carbon, to the solution is carried out after adding the at least one acid to the solution. Alternatively, adding said adsorbing agent, preferably active carbon, to the solution may be carried out before adding the at least one acid to the solution. With other words, the order of steps S12 and S14 may be changed and the order thereof is not fixed. Yet if the order is first setting of the pH below 7 to the desired pH value and then adding one or more adsorbing agents, preferably active carbon, will generate the best results with respect to protein removal and decolourization. In a preferred embodiment addition of the at least one acid antedates the addition of the at least one adsorbing agent, preferably active carbon.

In a preferred embodiment of the methods of the invention, the steps S12 and S14 are both performed and in the order S12 followed by S14.

The method then proceeds with first membrane filtration, preferably a micro- or ultrafiltration in a further step (Fig. 1, step S16) including a time suitable for the adhesion of colour components to the one or more adsorbing agents before the separation. The first membrane filtration is carried out so as to separate the biomass and the one or more adsorbing agents from the solution comprising the at least one aroma compound, and optionally at least one disaccharide and/or at least one monosaccharide, and by this removing the biomass and also reducing the colour components and protein in the resulting solution also called permeate comprising the aroma compounds, and optionally disaccharides and/or monosaccharides. Basically, step S16 includes microfiltration or ultrafiltration. However, as there is a smooth transition between microfiltration and ultrafiltration and both can be used by the skilled artisan to the purpose of separating biomass, adsorbing agent and protein on one side and the permeate containing the bulk of the desired one or more aroma compounds, one or more disaccharides and / or one or more monosaccharides on the other side. The filtration in step S16 may also be an ultrafiltration as an alternative to microfiltration. Said microfiltration or ultrafiltration is preferably carried out as cross-flow microfiltration or cross-flow ultrafiltration to improve membrane performance and reduce membrane abrasion. The details of the filtration in step S16 will be explained below. Said cross-flow microfiltration or cross-flow ultrafiltration includes a cross-flow speed in the range of 0.5 m/s to 6.0 m/s, preferably in the range of 2.0 m/s to 5.5 m/s and more preferably in the range of 3.0 m/s to 4.5 m/s, such as 4.0 m/s. In one embodiment the cross-flow speed is equal to or below 3.0 m/s, preferably between and including 1.0 and 2.0. One advantageous of the inventive method, use and the apparatus of the invention is that lower cross-flow speeds can be used to achieve good separation preferably of protein components of the solution from any aroma compounds, disaccharides or monosaccharides. Thus, energy and equipment cost can be reduced, wear and tear on equipment and abrasion of the filtration membrane are also reduced. Said first membrane filtration, preferably microfiltration or ultrafiltration, is carried out at a temperature of the solution in the range of 8 °C to 55 °C, preferably in the range of 10 °C to 50 °C and more preferably in the range of 30 °C to 40 °C, such as 38°C. Said microfiltration or ultrafiltration is carried out by means of a ceramic or polymeric microfiltration membrane or ceramic ultrafiltration membrane having a pore size in the range of 20 nm to 800 nm, preferably in the range of 40 nm to 500 nm and more preferably in the range of 50 nm to 200 nm, such as 100 nm. Said ceramic material is or has at least one layer of at least one ceramic material selected from the group consisting of: Titanium dioxide (TiO₂), Zirconium dioxide (ZrO₂), Silicon carbide (SiC) and Aluminium oxide (Al₂O₃). Alternatively, said microfiltration or ultrafiltration is carried out by means of a polymeric microfiltration membrane or polymeric ultrafiltration membrane having a cut-off in the range of 10 kDa to 200 nm, preferably in the range of 50 kDa to 200 nm and more preferably in the range of 50 kDa to 100nm. Said polymeric material is at least one polymeric material selected from the group consisting of: polyethersulfone, polysulfone, polypropylene, polyvinylidene fluoride, polyacrylonitrile, polyvinylidene fluoride. Said first membrane filtration, preferably microfiltration or ultrafiltration, is carried out after a predetermined time after the adsorbing agent, preferably active carbon, has been added to the solution. Thus, ensures adhesion of colour components. Typically, the time needed for mixing of the solution with the added adsorbing agent until a homogenous distribution of the adsorbing agent, preferably active carbon, in the solution has been reached may suffice to allow for the adhesion of the colour components, yet a longer incubation time can be used to maximize this. In one embodiment, said predetermined time is at least 2 min, preferably at least 10 min and more preferably at least 20 min such as 25 min or 30 min.

In one embodiment, the method of the invention typically then proceeds with a second membrane filtration step (Fig. 1, step S18). Preferably an ultrafiltration of the solution comprising aroma compounds obtained by the first membrane filtration of step S16 is carried out. In other words, an ultrafiltration of the permeate derived from the first membrane filtration in step S16 is carried out. Preferably, said second membrane filtration, preferably ultrafiltration, is carried out by means of an ultrafiltration membrane having a cut-off in the range of 1.5 kDa to 10 kDa, preferably in the range of 2 kDa to 10 kDa and more preferably in the range of 4 kDa to 5 kDa. In a particularly preferred embodiment, membranes with a cut-off of 4 kDa or 5 kDa are suitable. Said ultrafiltration membrane is at least partially made of a polymeric material. Said polymeric material is at least one polymeric material selected from the group consisting of: polyethersulfone, polyacrylonitrile, cellulose acetate. Said second membrane filtration, preferably ultrafiltration, is carried out at a temperature of the solution being in the range of 5 °C to 15 °C, preferably in the range 8 °C to 13 °C and more preferably in the range 8 °C to 12 °C, such as 10 °C.

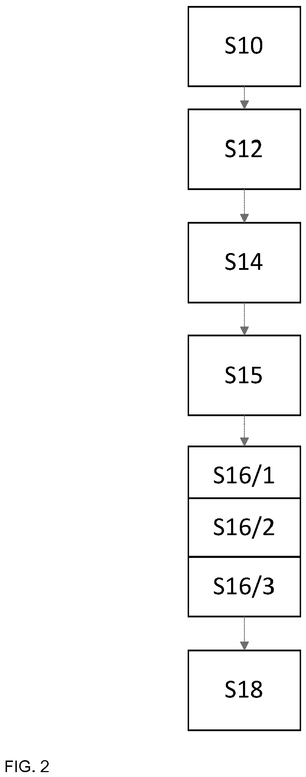

Fig 2 displays the sequence of steps of the inventive methods with the time suitable for the adhesion of colour components to the one or more adsorbing agents before the separation shown as a separate step (S15 in figure 2). Such a separate incubation step may be favorable when long times for sufficient adhesion of the undesired compounds to the adsorbing agent are required. Further, figure 2 depicts for the first membrane filtration (which is S16 in figure 1) as a step with three parts; the three steps of first membrane filtration being first diafiltration, concentrating and then optionally a second diafiltration. These are shown as S16/1, S16/2 and S16/3, respectively, in figure 2. The other steps are as in figure 1.

For the avoidance of doubt, any reference to the protein content of the solution or the permeate or retentate is referring to free protein in the solution / permeate / retentate, i.e. the protein found extracellularly and not the protein contained in the biomass if any. During fermentation and also subsequent handling and membrane filtrations, protein may be liberated from biomass and then be considered free protein.

For the avoidance of doubt, any reference to the at least one aroma compound, in the solution or the permeate or retentate is referring to the at least one aroma compound not bound in the solution / permeate / retentate, i.e. the at least one aroma compound, found extracellularly and not the ones contained in the biomass if any. During fermentation and also subsequent handling and membrane filtrations, the at least one aroma compound may be liberated from biomass and then be considered not bound aroma compound in the solution.

In a preferred embodiment, the step of carrying out first membrane filtration, preferably a microfiltration or ultrafiltration, so as to separate the biomass from the solution comprising the at least one aroma compound, is to be understood as a step of separating the biomass from the at least one aroma compoundwherein the majority of the at least one aroma compound is found in the permeate of the first membrane filtration following the separation of biomass.

In a preferred embodiment, the first membrane filtration is followed by an ultrafiltration, then optionally followed by a nanofiltration, ion exchange and/or reverse osmosis.

For the easier storage and transport, it is often desirable to have the fine chemicalsuch as an aroma compound in solid form rather than in solution. Hence, in a preferred embodiment the inventive method as a final step has the removal of the desired fine chemical(s) for example aroma compound(s) like Vanillin from the solution. This may be done by crystallisation for example but not limited to crystallisation with the help of one or more solvents such as but not limited to short chain alcohols (e.g. methanol, ethanol, propanol, butanol) and / or organic acids, preferably food-grade organic acids such as but not limited to acetic acid and/or propionic acid. Alternatively, solidification may be achieved in said final step of the inventive method by spray-drying or any other method for removal of water or solvent from the desired aroma compound to a suitable dryness of the aroma compound. Also, such steps of removing the aroma compound from the solution may be employed before said final step. For example the inventive method encompasses steps of crystallisation or spray drying followed by re-dissolving the aroma compound to create a new solution, optional other purification steps or repetitions of the removal from solution and re-dissolving of the aroma compound to form a new solution and then as a final step removal from the solution again.

Sometimes the desired fine chemical is present as a liquid at room temperature and preferably used as liquid, for example oil and the like; hence In an alternative embodiment, a solidification step is not needed for such fine chemical products. A solvent removal may be done for example.

In one embodiment, the fermentation broth is a fermentation broth from a two-phase fermentation process for example as disclosed in the international patent application published as WO2015002528, and more preferably the fermentation broth used in the current invention is one or more water-immiscible organic solvents used in the two-phase fermentation process.

Summarizing, the present invention includes the following embodiments, wherein these include the specific combinations of embodiments as indicated by the respective interdependencies defined therein.

### Further embodiments

Embodiment 1: A method for separating biomass from a solution comprising biomass and at least one aroma compound, and optionally at least one disaccharide and/or at least one monosaccharide, comprising the steps of:
a. providing the solution comprising biomass and at least one aroma compound, and optionally at least one disaccharide and/or at least one monosaccharide,
b. if the pH value is equal to or above pH 7.0, lowering the pH value of the solution below 7.0 by adding at least one acid to the solution comprising biomass and the at least one aroma compound, and optionally at least one disaccharide and/or at least one monosaccharide,
c. adding one or more adsorbing agents to the solution comprising biomass and at least one aroma compound, and optionally at least one disaccharide and/or at least one monosaccharide,
d. Optionally an incubation step sufficient for the one or more adsorbing agents to bind the colour components in the solution, and
e. carrying out first membrane filtration, preferably a microfiltration or ultrafiltration, to the effect that the biomass and the one or more adsorbing agents are separated from the solution comprising the at least one aroma compound, and optionally at least one disaccharide and/or at least one monosaccharide.

Embodiment 1A: A method for separating biomass from a solution comprising biomass and at least one aroma compound, and optionally at least one disaccharide and/or at least one monosaccharide, comprising the steps of:
a. providing the solution comprising biomass and at least one aroma compound, and optionally at least one disaccharide and/or at least one monosaccharide,
b. optionally adding one or more adsorbing agents to the solution
c. setting the pH value of the solution below 7.0, preferably below 5.5, more preferably below 5.0 and even more preferably to pH 4.5 or below, by adding at least one acid to the solution comprising biomass and the at least one aroma compound, and optionally at least one disaccharide and/or at least one monosaccharide,
d. adding one or more adsorbing agents to the solution comprising biomass and at least one aroma compound, and optionally at least one disaccharide and/or at least one monosaccharide in an amount suitable to remove colour components and the majority of the extracellular protein in the solution,
e. Optionally an incubation step sufficient for the one or more adsorbing agents to bind the colour components in the solution, and
f. carrying out first membrane filtration, preferably a microfiltration or ultrafiltration, to the effect that the biomass is separated from the solution comprising the majority of at least one aroma compound, and optionally at least one disaccharide and/or at least one monosaccharide.

Embodiment 1B: A method for separating biomass from a solution comprising biomass and at least one aroma compound, and optionally at least one disaccharide and/or at least one monosaccharide, comprising the steps of:
a. providing the solution comprising biomass and at least one aroma compound, and optionally at least one disaccharide and/or at least one monosaccharide,
b. lowering the pH value of the solution below 7.0 by adding at least one acid to the solution comprising biomass and the at least one aroma compound, and optionally at least one disaccharide and/or at least one monosaccharide,
c. adding one or more adsorbing agents to the solution comprising biomass and at least aroma compound, and optionally at least one disaccharide and/or at least one monosaccharide,
d. Optionally an incubation step sufficient for the one or more adsorbing agents to bind the colour components in the solution, and
e. carrying out first membrane filtration, preferably a microfiltration or ultrafiltration, so as to separate the biomass from the solution comprising the at least one aroma compound, and optionally at least one disaccharide and/or at least one monosaccharide.

Embodiment A1: An apparatus comprising
i. a solution containing biomass, at least one adsorbing agent, preferably active carbon and at least one aroma compound and optionally at least one disaccharide and/or at least one monosaccharide, wherein the pH value of the solution is below 7,
ii. a first filtration membrane, preferably a microfiltration or an ultrafiltration membrane,
iii. means to carry out a first membrane filtration across said first filtration membrane, preferably a microfiltration or ultrafiltration, to generate a permeate containing the bulk of the aroma compounds, disaccharides and or monosaccharides, and
iv. means to separate the permeate of the first membrane filtration from the solution as described in i. above,
   and further optionally comprising:
v. means to transport said permeate of the first membrane filtration to a second filtration membrane,
vi. means to adjust the temperature of the permeate to a temperature below 20 ° C,
vii. a second filtration membrane, preferably an ultrafiltration membrane,
viii. means to carry out a second membrane filtration, preferably a ultrafiltration, at a temperature below 20 ° C, and
ix. means to keep separate the permeate of the second membrane filtration from the permeate of the first membrane filtration,
wherein the surfaces of the parts of the apparatus that are in contact with the solution or any of the permeates are made of material suitable for the production of food and are tolerant to pH values as low as pH 3.5.

Embodiment A2: An apparatus comprising
i. a vessel holding a solution containing biomass and at least one aroma compound and optionally at least one disaccharide and/or at least one monosaccharide
ii. means to adjust the temperature of said solution to a temperature between 5°C and 70°C;
iii. a measuring system to measure the pH value of the solution in the vessel;
iv. means to set the pH value of the solution to a value below 7.0, preferably a target pH value lower than 5.5, wherein preferably the means to set the pH are suitable for the addition of at last one acid,
v. Means to add at least one adsorbing agent, preferably active carbon, to the solution,
vi. Means to generate an essentially homogenous distribution of the adsorbing agent in the solution
vii. a first filtration membrane, preferably a microfiltration or an ultrafiltration membrane,
viii. means to carry out with the help of said first filtration membrane a first membrane filtration, preferably a microfiltration or ultrafiltration, of the solution with a pH value below 7.0 and containing biomass, at least one adsorbing agent, preferably active carbon and at least one aroma compound and optionally at least one disaccharide and/or at least one monosaccharide, and wherein the means are suitable to generate a permeate containing the bulk of the aroma compounds, disaccharides and / or monosaccharides, and
ix. means to collect, transport and optionally store the permeate of the first membrane filtration from the solution with a pH value below 7.0 and containing biomass, at least one adsorbing agent, preferably active carbon and at least one aroma compound and optionally at least one disaccharide and/or at least one monosaccharide,
   and further optionally comprising:
x. means to transport said permeate of the first membrane filtration to a second filtration membrane,
xi. means to adjust the temperature of the first permeate to a temperature below 20°C,
xii. a second filtration membrane, preferably an ultrafiltration membrane,
xiii. means to carry out a second membrane filtration, preferably a ultrafiltration, at a temperature below 20° C, and
xiv. means to separate the permeate of the second membrane filtration from the permeate of the first membrane filtration,
wherein the surfaces of the parts of the apparatus that are in contact with the solution or any of the permeates are made of material suitable for the production of food and are tolerant to pH values as low as pH 3.5.

### Embodiment B1

Method for reducing wear and tear on and/or energy consumption of membrane filtration equipment used in the separation of biomass from a solution comprising at least one aroma compound, and optionally at least one disaccharide and / or at least one monosaccharide, wherein the method comprises these steps:
a. providing the solution comprising biomass and saccharides,
b. if the pH value is pH 7.0 or higher lowering the pH value of the solution below 7 by adding at least one acid to the solution comprising biomass and comprising at least one aroma compound, and optionally at least one disaccharide and / or at least one monosaccharide,
c. adding one or more adsorbing agents to the solution comprising biomass and aroma compound,
d. Optionally as required an incubation step sufficient for the one or more adsorbing agents to bind the colour components in the solution, and
e. carrying out first membrane filtration so as to separate the biomass from the solution comprising the comprising at least one aroma compound, and optionally at least one disaccharide and / or at least one monosaccharide at cross-flow speeds of no more than 3 m/s.

Embodiment 2: The method according to any of the embodiments 1, 1A, 1B or B1, or the apparatus according to embodiment A1 or A2, wherein the adsorbing agent is active carbon.

Embodiment 3: The method or apparatus according to any of the previous embodiments, wherein the pH value of the solution is lowered to a pH value in the range of 3.0 to 5.5, preferably the range of 3.5 to 5 and more preferably the range of 4.0 to 4.5.

Embodiment 4: The method or apparatus according to any of the previous embodiments, wherein said at least one acid is an acid selected from the group consisting of H₂SO₄, H₃PO₄, HCl, HNO₃ and CH₃CO₂H.

Embodiment 5: The method or apparatus according to any of the previous embodiments, wherein said adsorbing agent, preferably active carbon, is added in an amount in the range of 0.5 % to 3 % by weight, preferably in the range of 0.75 % to 2.5 % by weight or from and including 0.5 % to and including 1.5 % by weight and more preferably in the range of 1.0 % to 2.0 % by weight.

Embodiment 6: The method or apparatus according to any of the previous embodiments, wherein said adsorbing agent, preferably active carbon, is added as a powder having a particle size distribution with a diameter d50 in the range of 2 µm to 25 µm, preferably in the range of 3 µm to 20 µm and more preferably in the range of 3 µm to 7 µm.

Embodiment 7: The method or apparatus according embodiment 6, wherein said adsorbing agent, preferably active carbon, is added as a suspension of the adsorbing agent powder in water.

Embodiment 8: The method or apparatus according to any of the previous embodiments, wherein adding said adsorbing agent, preferably active carbon, to the solution is carried out when the pH value of the solution is below 7, and while at least one acid continues to be added to the solution or after adding the at least one acid to the solution has been completed.

Embodiment 9: The method or apparatus according to any of the previous embodiments except embodiment 8, wherein adding said adsorbing agent, preferably active carbon, to the solution is carried out before adding the at least one acid to the solution.

Embodiment 10: The method or apparatus according to any of the previous embodiments, wherein said solution comprising biomass and one or more aroma compounds, one or more disaccharides and / or one or more monosaccharides is obtained by cultivation of one or more types of cells, preferably bacteria or yeast, more preferably bacteria, even more preferably genetically modified Escherichia coli, in a cultivation medium, preferably a cultivation medium comprising at least one carbon source, at least one nitrogen source and inorganic nutrients.

Embodiment 11: The method or apparatus according to any of the previous embodiments , wherein providing the solution comprising biomass and at least one aroma compound, one or more disaccharides and / or one or more monosaccharides includes preparing said solution by means of microbial fermentation.

Embodiment 12: The method or apparatus according to any of the previous embodiments except embodiment B1, wherein said first membrane filtration is carried out as cross-flow microfiltration or cross-flow ultrafiltration.

Embodiment 13: The method or apparatus according to embodiment 12, wherein said cross-flow microfiltration or cross-flow ultrafiltration includes a cross-flow speed in the range of 0.5 m/s to 6.0 m/s, preferably in the range of 2.0 m/s to 5.5 m/s and more preferably in the range of 2.2 m/s to 4.5 m/s and even more preferably in the range of 2.5 to 4.5.

Embodiment 14: The method or apparatus according to any of the previous embodiments, wherein said first membrane filtration is carried out at a temperature of the solution in the range of 8 °C to 55 °C, preferably in the range of 10 °C to 50 °C and more preferably in the range of 30 °C to 40 °C.

Embodiment 15: The method or apparatus according to any of the previous embodiments, wherein said first membrane filtration is carried out by means of a ceramic microfiltration membrane or ceramic ultrafiltration membrane having a pore size in the range of 20 nm to 800 nm, preferably in the range of 40 nm to 500 nm and more preferably in the range of 50 nm to 200 nm.

Embodiment 16: The method or apparatus according to embodiment 15, wherein said ceramic material is at least one ceramic material selected from the group consisting of: TiO₂, ZrO₂, SiC and Al₂O₃.

Embodiment 17: The method or apparatus according to any of the previous embodiments, wherein said first membrane filtration is carried out by means of a polymeric microfiltration membrane or polymeric ultrafiltration membrane having a cut-off in the range of 10 kDa to 200 nm, preferably in the range of 50 kDa to 200 nm and more preferably in the range of 50 kDa to 100nm.

Embodiment 18: The method or apparatus according to embodiment 17, wherein said polymeric material is at least one polymeric material selected from the group consisting of: polyethersulfone, polysulfone, polypropylene, polyvinylidene fluoride, polyacrylonitrile, polyvinylidene fluoride.

Embodiment 19: The method or apparatus according to any of the previous embodiments, wherein said first membrane filtration is carried out after a predetermined time after the adsorbing agent, preferably active carbon, has been added to the solution.

Embodiment 20: The method or apparatus according to embodiment 19, wherein said predetermined time is at least 2 min, preferably at least 10 min and more preferably at least 20 min.

Embodiment 21: The method of any of the previous embodiments, wherein the first membrane filtration comprises preferably two, more preferably three steps: a first diafiltration step, a concentrating step and optionally a second diafiltration step, each as disclosed in detail in this application.

Embodiment 22: The method according to any one of the previous embodiments, further comprising carrying out a second membrane filtration, of the solution comprising at least one aroma compound, one or more disaccharides and / or one or more monosaccharides obtained by the first membrane filtration, preferably an ultrafiltration of the permeate of the first membrane filtration.

Embodiment 23: The method according to embodiment 22, wherein said second membrane filtration is an ultrafiltration and is carried out by means of an ultrafiltration membrane having a cut-off in the range of 1.0 kDa to 10 kDa, preferably in the range of 2 kDa to 10 kDa and more preferably in the range of 4 kDa to 5 kDa.

Embodiment 24: The method according to embodiment 23, wherein said ultrafiltration membrane is at least partially made of a polymeric material.

Embodiment 25: The method according to embodiment 24, wherein said polymeric material is at least one polymeric material selected from the group consisting of: polyethersulfone, polyacrylonitrile, cellulose acetate.

Embodiment 26: The method according to any one of embodiments 22 to 25, wherein said second membrane filtration, preferably ultrafiltration, is carried out at a temperature of the solution being in the range of 5 °C to 15 °C, preferably in the range 8 °C to 13 °C and more preferably in the range 8 °C to 12 °C.

Embodiment 27: The method according to any one of embodiments 22 to 26, wherein the solution comprising aroma compound obtained by the first membrane filtration is brought to a temperature of below 20 °C before and preferably maintained a temperature of below 20 °C during said second membrane filtration.

Embodiment 28: The method or apparatus according to any one of the previous embodiments, wherein said at least one aroma compound Vanillin.

### Figures:

Figure 1 shows a block diagram of a method for separating biomass from a solution comprising biomass and at least one aroma compound according to the present invention.
Figure 2 displays in a block diagram the sequence of steps of the inventive methods when a separate incubation step S15 for the adsorbing agent and the three sub steps S16/1 to S16/3 as explained above.

### Examples

The method according to the present invention will be described in further detail below. Whatsoever, the Examples shall not be construed as limiting the scope of the invention.

### Example 1

A fermentation broth as a complex solution comprising biomass and at least one aroma compound is being prepared by standard methods. The pH value thereof is lowered to 4 ± 0.1 by means of adding 10% sulphuric acid. Thereafter, a 30% suspension of active carbon Carbopal Gn-P-F (Donau Carbon GmbH, Gwinnerstraße 27-33, 60388 Frankfurt am Main, Germany), which is food safe, is added and stirred for 20 min.

The thus prepared solution is supplied to the process apparatus, a semi-automatic MF lab unit from Sartorius AG, Otto-Brenner-Str. 20, 37079 Goettingen, Germany, modified for the purpose, and heated to 37 °C in a circulating manner with closed permeate. For separation purposes, the process apparatus includes a ceramic mono channel element (from Atech Innovations GmbH, Gladbeck, Germany) having an outer diameter of 10mm, an inner diameter of 6 mm, a length of 1.2 m and a membrane made of Al₂O₃ having a pore size of 50 nm. As soon as the circulation of the solution is running and the solution comprises the target temperature of 37 °C, the discharging of the permeate is started and the control of the trans membrane pressure is activated.

After terminating of the inventive method, the process apparatus is stopped, the concentrate is disposed, and the process apparatus is being cleaned. Cleaning is carried out by means of 0.5 % to 1% NaOH at a temperature of 50 °C to 80 °C, wherein the NaOH is subsequently removed by purging.

In a preferred embodiment, the first membrane filtration of the inventive methods includes three steps as will be explained in further detail below. The first step includes a first diafiltration having a factor of 0.5 (amount of diafiltration water = starting amount of fermentation broth x diafiltration factor). During diafiltration, the amount of water added is identical to the amount of permeate discharged. The first step is a continuing step and the volume in the feed vessel is thus kept constant. The second step includes concentrating of the fermentation broth with the factor 2 by stopping the feed of diafiltration water and the level will decrease down to the target value (target value = volume or mass at the beginning of the fermentation broth / concentrating factor). Subsequently, the third step includes a second diafiltration. The permeates collected during these three steps are typically combined to form the permeate referred to in the tables below. By means of these three steps a lower dilution of the product within the permeate and an increased yield are realized. By increasing the factor of the second diafiltration, the yield may even be increased.

The following analytical methods are been carried out.
- HPLC or GC or GC-MS for the determination of the product, i.e. aroma compounds, and secondary components
- Drying balances for measuring the dry content
- APHA for measuring the colour using standard methods, for example DIN EN ISO 6271
- Bradford protein assay for measuring the concentration of protein.

Some experiments are made with different fermentation broths as these may not be stored over a longer period of time. In order to be able to determine whether the method correctly works and provides the announced advantages, experiments are made:
- without adjustment of pH value and without adding active carbon,
- without adjustment of pH value and with adding active carbon,
- after adjustment of pH value and without adding active carbon,
- after adjustment of pH value and with adding active carbon,
- after adding active carbon and then adjustment of pH value.

Hereinafter, the following abbreviations are used:
- AC = Active Carbon
- UF = Ultrafiltration
- DP = Pressure drop along the module (p_{feed}-pᵣₑₜₑₙₜₐₜₑ)
- Flux = Permeate flow rate per m² and hour (l/m²h)
- Cross-flow velocity = linear speed of the suspension in membrane channels (m/s)
- Membrane load = amount of permeate produced by 1m² of membrane area (m³/ m²)

Further, regarding the liquid separation, the following symbols and explanations are used.

| **Symbol** | **Meaning** | **Unit** | **Definition** |
|---|---|---|---|
| *Letters* | • | | |
| *CF* | Concentration factor • | - | m_{*R*,*t*}=0/ *m*_{R} |
| *DF* | Diafiltration factor | - | *m*_{P}/m_{*R*,*t*}=0 |
| *J* | • Flux | LMH = L m⁻² h⁻¹ | |
| *m* | Mass | kg | |
| | • | | |
| *p* | Pressure • | bar | |
| *R* | Retention • | - | *1*- *c*ₚₑᵣₘₑₐₜₑ/*c*ᵣₑₜₑₙₜₐₜₑ |
| *TMP* | Trans-membrane pressure | bar | (*p*_{feed} + *p*ᵣₑₜₑₙₜₐₜₑ)/2 - *p*ₚₑᵣ₋ₘₑₐₜₑ |

The membrane performance depends on the pH value and active carbon.

Different batches of fermentation broth originating from fermentations with varying parameters resulting in a solution with differing colour components and different aroma compound of the solution demonstrate the broad applicability of the methods of the invention.

Cited Literature
- EP 2 379 708
- EP1081212
- WO2015002528
- US9115377
   US6133033- CN105132472
- CN105132472
- WO2007099230

## Claims

1. A method for separating biomass from a solution comprising biomass and at least one aroma compound, comprising:
- providing the solution comprising biomass and aroma compounds;
- setting the pH value of the solution below 7 by adding at least one acid to the solution comprising biomass and the at least one aroma compound;
- adding at least one adsorbing agent, preferably active carbon, to the solution comprising biomass and aroma compounds; and
- carrying out a first membrane filtration, preferably a microfiltration or an ultrafiltration, so as to separate the biomass from the solution comprising at least one aroma compound.

2. The method according to claim 1, wherein the pH value of the solution is lowered to a pH value in the range of 3.0 to 5.5, preferably the range of 3.5 to 5 and more preferably the range of 4.0 to 4.5.

3. The method according to any one of claims 1 to 2, wherein said at least one acid is an acid selected from the group consisting of H₂SO₄, H₃PO₄, HCI, HNO₃ and CH₃CO₂H.

4. The method according to any one of claims 1 to 3, wherein said adsorbing agent, preferably active carbon, is added in an amount in the range of 0.3 % to 3 % by weight, preferably in the range of 0.4 % to 2.5 % by weight and more preferably in the range of 0.5 % to 1.5 % by weight.

5. The method according to any one of claims 1 to 4, wherein said adsorbing agent, preferably active carbon, is added as a powder having a particle size distribution with a diameter d50 in the range of 2 µm to 25 µm, preferably in the range of 3 µm to 20 µm and more preferably in the range of 3 µm to 7 µm.

6. The method according to claim 5, wherein said adsorbing agent, preferably active carbon, is added as a suspension of the powder in water.

7. The method according to any one of claims 1 to 6, wherein said first membrane filtration is carried out as cross-flow microfiltration or cross-flow ultrafiltration.

8. The method according to claim 7, wherein said cross-flow microfiltration or cross-flow ultrafiltration includes a cross-flow speed in the range of 0.5 m/s to 6.0 m/s, preferably in the range of 2.0 m/s to 5.5 m/s and more preferably in the range of 3.0 m/s to 4.5 m/s.

9. The method according to claim 7, wherein said cross-flow speed is equal to or below 3 m/s and preferably for polymeric membranes equal to or below 1.7 m/s

10. The method according to any one of claims 1 to 9, wherein said first membrane filtration is carried out at a temperature of the solution in the range of 8 °C to 55 °C, preferably in the range of 10 °C to 50 °C and more preferably in the range of 30 °C to 40 °C.

11. The method according to any one of claims 1 to 10, wherein said first membrane filtration is carried out by means of a ceramic microfiltration or ultrafiltration membrane having a pore size in the range of 20 nm to 800 nm, preferably in the range of 40 nm to 500 nm and more preferably in the range of 50 nm to 200 nm, or wherein said first membrane filtration is carried out by means of a polymeric microfiltration membrane or polymeric ultrafiltration membrane having a cut-off in the range of 10 kDa to 200 nm, preferably in the range of 50 kDa to 200 nm and more preferably in the range of 50 kDa to 100nm.

12. The method according to any one of claims 1 to 11, further comprising carrying out a second membrane filtration with the solution comprising aroma compounds obtained by the first membrane filtration, preferably an ultrafiltration with a membrane having a lower cut-off than the membrane of the first membrane filtration.

13. The method according to claim 12, wherein said second membrane filtration is an ultrafiltration and is carried out by means of an ultrafiltration membrane having a cut-off in the range of 1.5 kDa to 10 kDa, preferably in the range of 2 kDa to 10 kDa and more preferably in the range of 4 kDa to 5 kDa.

14. The method according to any one of claims 12 to 13, wherein said second membrane filtration is carried out at a temperature of the solution being in the range of 5 °C to 15 °C, preferably in the range 8 °C to 13 °C and more preferably in the range 8 °C to 12 °C.

15. The method according to any one of claims 1 to 14, wherein said at least one aroma compound comprises at least one polar aroma compound, preferably selected from furaneol, benzoic acid, phenylethanol, raspberry ketone, pyrazines, vanillin, vanillyl alcohol and vanilla glycoside, and more preferably selected from vanillin, vanillyl alcohol and vanilla glycoside.
